# EUROPEAN PATENT SPECIFICATION

(11) **EP 1 194 072 B1**
(45) Date of publication and mention of the grant of the patent: **26.09.2007**
(21) Application number: 00944546.1
(22) Date of filing: 18.06.2000
(51) Int. Cl.: A61B 17/00, A61B 18/00, A61L 27/00, A61L 31/00

(54) **MICRO TOOLS**
MIKROINSTRUMENTE
MICRO INSTRUMENTS

(30) Priority: 21.06.1999 SE 9902348
(43) Date of publication of application: 10.04.2002
(73) Proprietor: MicroMuscle AB, 582 16 Linköping (SE)
(72) Inventor: INGANÄS, Olle, S-582 46 Linköping (SE); JAGER, Edvin, S-584 32 Linköping (SE); SELBING, Anders, S-589 35 Linköping (SE)
(74) Representative: Willquist, Bo Lorentz
(86) International application number: PCT/SE2000/001286
(87) International publication number: WO 2000/078222

(56) References cited:
- EP-A- 0 870 319
- US-A- 5 771 902

## Description

### Technical Field

This invention concerns a tool for biomedical surgery according to the pre-characterizing portion of claim 1. Such tools can be used to adapt, assemble, separate, fortify, dilate, close and hold biological structures inside the body during and after surgery. The tools may be stents, valves, clips, nets, knives, scissors, dilators, clamps, tweezers etc.

### Background

The use of microstructures to assemble, fortify or dilate biological structures inside the body during and after surgery can help the surgeon in a number of ways. The operation of electrically actuated tools can help the surgeon to simultaneously position, operate manually, and observe. By positioning the tool by hand and separately operating it through external control (i.e. footswitch, voice control, other software-control) a much higher degree of precision is expected. In microsurgery, this is an especially desired advantage.

The combination of microactuators and catheters are not well documented in the literature. A patent search reveals a few examples but none that describes the use of microactuators as tools housed inside a catheter, several examples of microactuators use to position a catheter are to be found in the following patents

| | |
|---|---|
| US5771902 . | Micromachined actuators/sensors for intratubular positioning/steering |
| US5819749 | Microvalve |
| WO9837816A1 | Microfabricated therapeutic actuators |
| WO9739688A2 | Method and apparatus for delivery of an appliance in a vessel |
| WO9739674A1 | Spring based multi-purpose medical instrument |
| US5855565 | Cardiovascular mechanically expanding catheter |

The preamble of claim 1 is based on US5771902.

Several mechanisms are suggested for the microactuators in these applications, found among shape memory alloys (including polymeric materials) and piezoelectric materials. The use of conjugated polymers in micromuscles is not documented for catheter tools.

To be able to apply, beforehand or during an invasive procedure, a tool of a required size and geometry - designed for the purpose of cutting, drilling, holding, dilating, suturing, adapting or supporting - from tools that, for example, could be introduced through, placed inside or located at the end of a catheter or needle, is another desired function, requiring development of microactuators.

### Summary of the Invention

The present invention provides a tool for biomedical surgery having the features of claim 1. Embodiments are set forth in the dependent claims, in the following description and in the appended drawings.

Our novelty and innovation therefore resides in the use of microactuators based on conjugated polymers being electrically operated and mounted in or on a catheter or needle, to be positioned with the help of the catheter, and then activating the microactuator structures carried on the needle. The microfabrication of such microactuators renders possible a number of geometries from 10 µm and larger, difficult to produce by mechanical production techniques. They may be produced by use of the method presented in WO-A-96/28841 and then mounted in or on the needle or catheter, or they might be produced by novel manufacturing methods. With the help of this invention, completely novel microsurgery tools are available.

### Brief Description of the Drawings

Figure 1A - 1C shows clips and clip arrays, where the clips are mounted in sequence, and area confined by a cylindrical housing, and where the activation of the outer most clip C1, opening up the clip to join the open structure W1, and then being set free by the simultaneous operation of C2, so as to be left at the structure W1, holding the structures together.
Figure 2 shows tubular tweezers, tweezers and knifes, based on microactuators. The indicated movement is driven by microactuators properly mounted and designed.
Figure 3A - 3B shows a neural connector, where a number of small fingers coil around a cylindrical nerve to make a tight hold the nerve. Two separate nerves are here joined with the help of a common neural connector, which would be desired for accomplishing regrowth of the nerves. In addition, small electrodes can be fashioned along with the microfingers, and be used to sense or excite nerve signals.
Figure 4A - 4C. An insertion devise, for making a temporally permanent hole through a membrane. The devise is housed in a catheter/cannula/needle and is inserted through the membrane so as to make the devise form a hole through the membrane.
Figure 5A - 5B show a stent device.

### Description of Embodiments

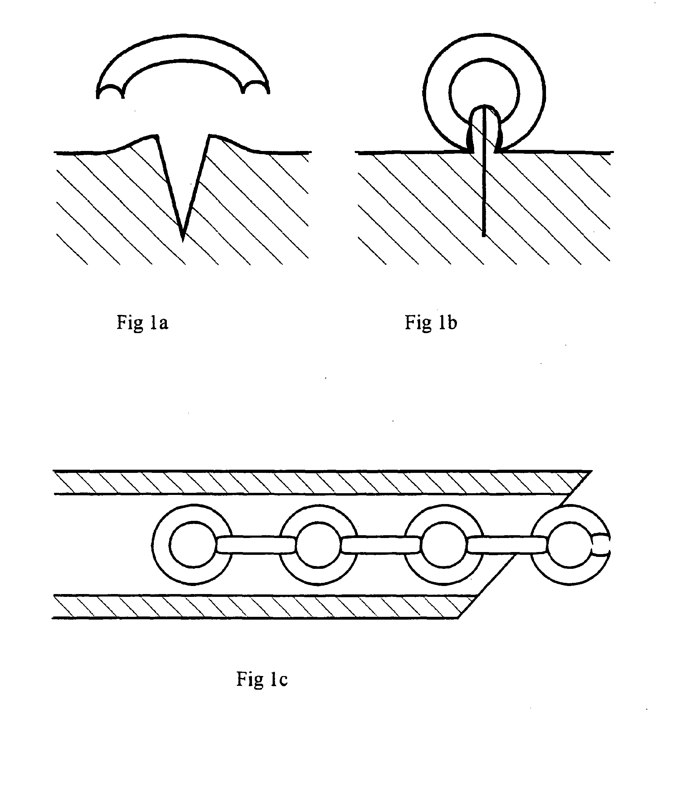

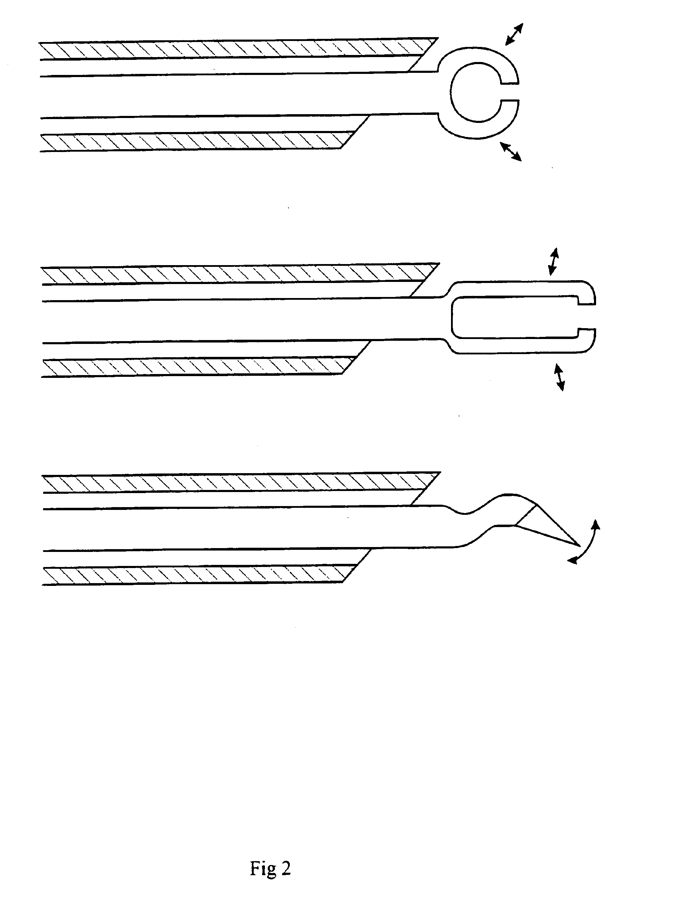

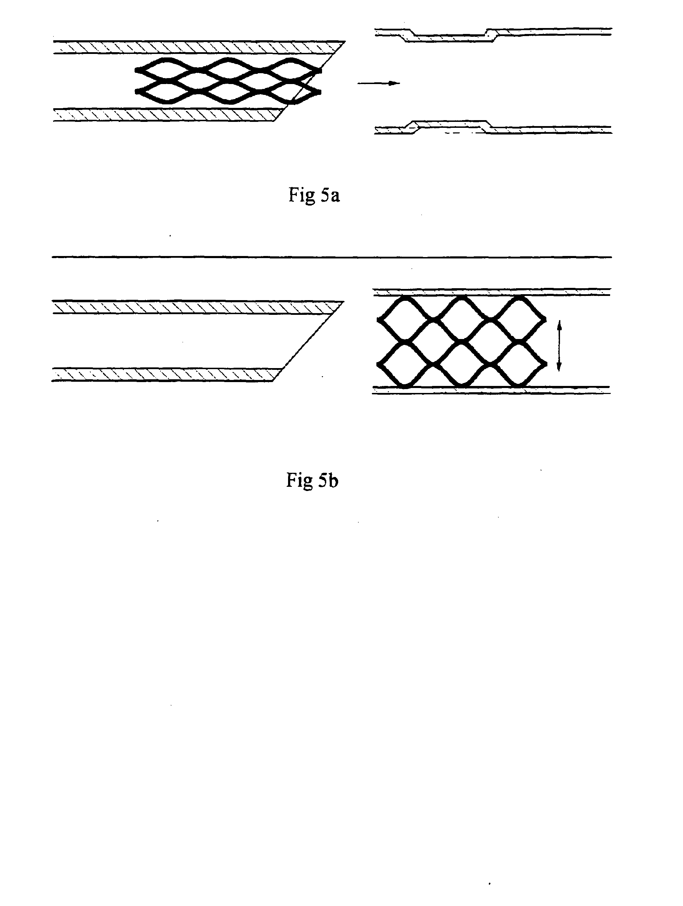

The application of structure in or introduced through a catheter or needle is of particular interest at the application of tools, which are to be left at the site after insertion, and which have to execute their function for some limited time duration. The first example here is that of clips for surgery, sub-millimeter to millimeter structures, which would be used to hold two separated biological structures joined, for example during a healing period (Fig.1A - 1C). -Another example is that of structures for controlling the flow through blood vessels. The simplest level is that of a clip used to prevent blood flow to a biological structure downstream in the blood flow. Such a clip, or series of clips, would be mounted and left to hold a firm grip on the blood vessel and thus to prevent the flow of blood. In Figure 2 is shown a series of structures suitable for constricting blood vessels.
The third example is at a somewhat more complex level with structures built in a geometry where they could be used inside or outside tube-like structures, as so called stents to dilate a stenotic area or to internally or externally fortify or join the structure(s) (Figure 5A and 5B). Stents are of particular interest since they are to be inserted inside the tube, then to be left there to expand a stenotic (examples: blood vessel, biliary duct) or to fortify a weak (examples: blood vessel with aneurysm, divided biliary duct) part of a tubular structure.

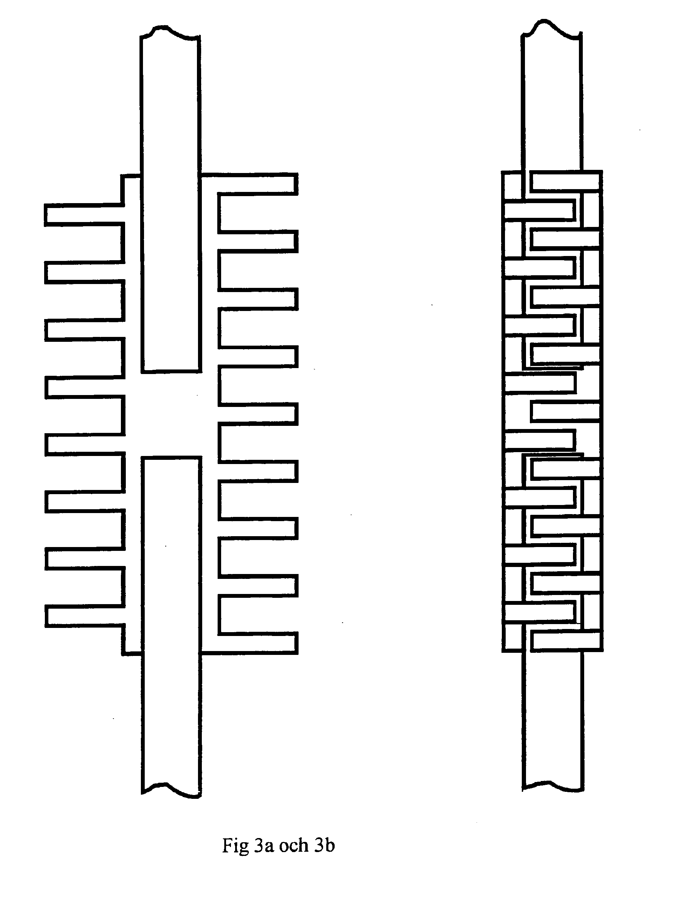

Arrays of fingers could be used to hold cylindrical objects, such as nerves and nerve fibers, or blood vessels. With the help of microactuators holding the structures (Fig. 3A - 3B), adjacent microstructures operating as neural sensing or activating electrodes, will enable recording signals from or activating nerves. This could be used as a synthetic neural connector, bridging a severed nerve or nerve fiber.

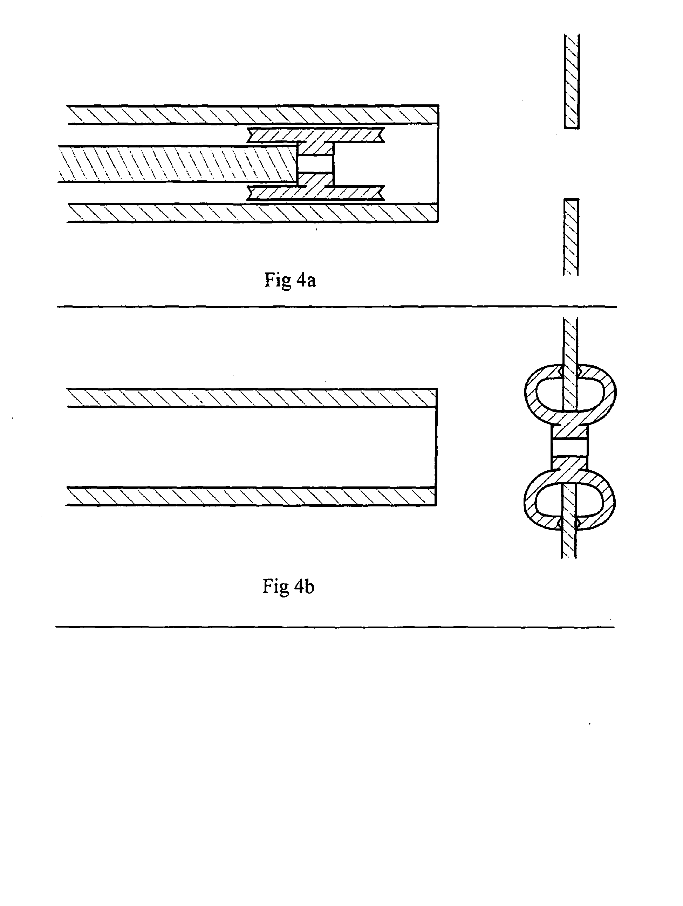

Elements with some temporary mechanical function could be inserted in membranes (Fig.4A-4C). Insertion devices of this kind could be used for mounting a hole through a membrane such as commonly used in ear surgery for pressure equilibration. Making these as microdevices will much decrease the effort to place and remove the inserted devices and to keep them in place during the desired time period.

Clips, stents, finger arrays and insertion devices, once applied, could be resorbable or permanent. They could express various degrees of stimulation of cell growth on its surfaces, various degrees of anti-thrombotic activity as well as different antibiotic activities. They can also be carriers of various biochemical or biological components.

The necessary elements to accomplish these functions are the electrochemically activated micromuscles, built by micromachining thin metal and polymer layers (Elisabeth Smela, Olle Inganäs and Ingemar Lundström: "Controlled Folding of Micron-size Structures", Science 268 (1995) pp.1735-1738) or only polymer layers. These actuators can be produced in sizes from micrometers to centimeters, and operate well in biological fluids such as blood plasma, blood, buffer and urine. They are therefore suitable tools for micro invasive surgery inside the body. The versatility of construction and the speed of response, as well as the force of these actuators render them as one of the best types of microactuators inside the body. An international patent covers one route of fabrication of such devices (Elisabeth Smela, Olle Inganäs and Ingemar Lundström: "Methods for the fabrication of micromachined structures and micromachined structures manufactured using such methods", WO 96/28841.

The production of individually actuated tool arrays render little difficulty beyond that of producing the individual tool; we have to see that electrical contacts are supplied to actuate each microactuator separately. This can be done by wiring the single microactuator, to be used as the working electrode; the catheter is then used as the counterelectrode, and will be able to supply all the charge that we ever need to actuate all those microactuators. As wires may easily be produced in width down to 10 µm with photolithography or with soft lithography, we will be able to put at least 50 microactuators along the tool array located in/on a needle of 1 mm width, with the simple philosophy of putting down parallel conductor wires. Should we need more, more elaborate addressing schemes might be needed.
Should a necessity for three electrode systems be found in any of the applications, microfabricated reference electrodes or macrosize reference electrodes carried on the catheter housing offers a solution for this problem.

Should the tool arrays be collectively addressed, and the tool array is designed to set free the outermost clip on actuation of all the clips, we will need a mechanism of confining the movements of all but the outermost clip. This is done by assembling the clip array into a cylindrical housing, preferably the catheter, prior to insertion in the body. The cylindrical housing is now confining the motion of microactuators, which search in vain to expand the strong metal casing on operation. When the outermost clip C1 is actuated, the clip is opened; likewise is the next-to-the outermost clip C2 partially free to move as it is protruding outside the cylindrical housing. Therefore the partial opening of C2 sets C1 free, as well as opens it up for subsequent spontaneous closing on the site to be clipped.

## Claims

1. A toll for biomedical surgery, wherein
the tool comprises a layered polymer micromuscle arranged to induce geometrical changes and movements via an electrochemically induced change of volume in at least one polymer layer, thereof **characterised in that**
the tool is mounted on a carrier having the form of a needle adapted to be inserted into a cannula or catheter, through which the tool is electrically actuatable via external means to induce a mechanical movement to act upon a biological structure.

2. A tool according to claim 1, wherein the layered polymer consists of a single layered polymer.

3. A tool according to claim 1, wherein the layered polymer consists of a bilayered polymer.

4. A tool according to claim 1, wherein the layered polymer consists of multilayered polymer and metal layers.

5. A tool according to one or more of claims 1-4, wherein the polymer micromuscles are built of layers, of which at least one is a conjugated polymer.

6. A tool according to claim 5, wherein the conjugated polymer is selected from the group consisting of pyrrole, aniline, thiophene, para-phenylene, vinylene, and phenylene polymers and copolymers, including substituted forms of the different monomers.

7. A tool according to claim 1, wherein the tool is built of bi-layered polymer, where the electrically activated volume change of said, at least one conjugated polymer is arranged to cause a bending of said bi-layer.

8. A tool according to claim 1, wherein the tool is built of multilayered polymer, where the electrically activated volume change of said, at least one conjugated polymer is arranged to cause a bending of said multilayer.

9. A tool according to one or more of claims 1-8, wherein the tool comprises means for providing the mechanical movement for positioning a biological structure.

10. A tool according to one or more of claims 1-8, wherein the tool comprises means for providing the mechanical movement for holding a biological structure.

11. A tool according to one or more of claims 1-8, wherein the tool comprises means for providing the mechanical movement for cutting a biological structure.

12. A tool according to one or more of claims 1-8, wherein the tool comprises means for providing the mechanical movement for dilating a biological structure.

13. A tool according to one or more of claims 1-8, wherein the tool comprises means for providing the mechanical movement for fortifying a biological structure.

14. A tool according to one or more of claims 1-8, wherein the tool comprises means for providing the mechanical movement for implanting a biological structure.

15. A tool according to one or more of claims 1-14, **characterized in that** the individual tool is a scissors.

16. A tool according to one or more of claims 1-14, **characterized in that** the individual tool is a knife, which is arranged on an actuator, being arranged for linear and/or angular movement.

17. A tool according to one or more of claims 1-14, **characterized in that** the individual tool is a sharp needle that is arranged on an actuator being arranged for linear and/or angular movement.

18. A tool according to one or more of claims 1-14, **characterized in that** the individual tool is a dilator.

19. A tool according to one or more of claims 1-14, **characterized in that** the individual tool is a clamp.

20. A tool according to one or more of claims 1-14, **characterized in that** the individual tool is a tweezers.

21. A tool array comprising a tool according to one or more of claims 1-8, wherein a number of identical tools are located on a tool array extending along a length of the cannula, catheter or needle, and wherein the actuation of a tool closest to the exit of the catheter is arranged to release a tool from the tool array and is arranged to leave it at the point of exit of the catheter for mounting the tool at/in some biological structure.

22. A tool array according to claim 21, wherein a number of identical tools are located on the tool array extending along the catheter or needle and where each tool is arranged to become individually actuated.

23. A tool array according to claim 21, wherein number of non-identical tools are located on the tool array extending along the catheter or needle and where each tool is arranged to become individually actuated.

24. A toll array according to anyone of claims 21-23, **characterized in that** the individual tool is a clip arranged to join biological tissues or tissue parts, and arranged to hold the said tissues or tissue parts to allow healing.

25. A tool array according to anyone of claim 21-23, **characterized in that** the individual tool is an expandable cylindrical object designed to be inserted, in a contracted state, into a biological tube, and arranged to become expanded to keep said tube in an expanded state or to join two or more biological tubes.

## Patentansprüche

1. Instrument für die biomedizinische Chirurgie, wobei das Instrument einen geschichteten Polymermikromuskel enthält, welcher angeordnet ist, um geometrische Veränderungen und Bewegungen mittels einer elektrochemisch induzierten Änderung des Volumens in mindestens einer Polymerschicht hiervon hervorzurufen,
**dadurch gekennzeichnet, dass** das Instrument auf einem Träger befestigt ist, welcher die Form einer zum Einbringen in eine Kanüle oder einen Katheter geeignete Nadel hat, durch welche das Instrument elektrisch ansteuerbar mittels externer Hilfsmittel ist, um eine mechanische Bewegung zur Einwirkung auf ein biologisches Gebilde zu erzeugen.

2. Instrument nach Anspruch 1, wobei das geschichtete Polymer aus einem einschichtigen Polymer besteht.

3. Instrument nach Anspruch 1, wobei das geschichtete Polymer aus einem zweischichtigen Polymer besteht.

4. Instrument nach Anspruch 1, wobei das geschichtete Polymer aus vielschichtigen Polymer- und Metallschichten besteht.

5. Instrument nach einem oder mehreren der Ansprüche 1 bis 4, wobei die Polymermikromuskeln aus Schichten aufgebaut sind, von denen mindestens eine ein konjugiertes Polymer ist.

6. Instrument nach Anspruch 5, wobei das konjugierte Polymer ausgewählt ist aus einer Gruppe, bestehend aus Pyrrol, Anilin, Thiophen, Paraphenylen, Vinylen und Phenylen Polymeren und Copolymeren, ersatzweise Gestaltungen der unterschiedlichen Monomere enthaltend.

7. Instrument nach Anspruch 1, wobei das Instrument aus einem zweischichtigen Polymer aufgebaut ist, wo die elektrisch angesteuerte Volumenänderung des besagten, mindestens einen konjugierten Polymer ausgeführt wird, um eine Biegung der besagten Doppelschicht zu erzeugen.

8. Instrument nach Anspruch 1, wobei das Instrument aus einem vielschichtigem Polymer aufgebaut ist, wo die elektrisch angesteuerte Volumenänderung des besagten, mindestens einen konjugierten Polymers ausgeführt wird, um eine Biegung der besagten Multischicht zu erzeugen.

9. Instrument nach einem oder mehreren der Ansprüche 1 bis 8, wobei das Instrument Hilfsmittel umfasst, um für die mechanische Bewegung zur Positionierung einer biologischen Struktur zu sorgen.

10. Instrument nach einem oder mehreren der Ansprüche 1 bis 8, wobei das Instrument Hilfsmittel umfasst, um für die mechanische Bewegung zum Halten einer biologischen Struktur zu sorgen.

11. Instrument nach einem oder mehreren der Ansprüche 1 bis 8, wobei das Instrument Hilfsmittel umfasst, um für die mechanische Bewegung zum Schneiden einer biologischen Struktur zu sorgen.

12. Instrument nach einem oder mehreren der Ansprüche 1 bis 8, wobei das Instrument Hilfsmittel umfasst, um für die mechanische Bewegung zum Weiten einer biologischen Struktur zu sorgen.

13. Instrument nach einem oder mehreren der Ansprüche 1 bis 8, worin das Instrument Hilfsmittel umfasst, um für die mechanische Bewegung zur Verstärkung einer biologischen Struktur zu sorgen.

14. Instrument nach einem oder mehreren der Ansprüche 1 bis 8, wobei das Instrument Hilfsmittel umfasst, um für die mechanische Bewegung zum Implantieren einer biologischen Struktur zu sorgen.

15. Instrument nach einem oder mehreren der Ansprüche 1 bis 14,
**dadurch gekennzeichnet, dass** das einzelne Instrument eine Schere ist.

16. Instrument nach einem oder mehreren der Ansprüche 1 bis 14,
**dadurch gekennzeichnet, dass** das einzelne Instrument ein Messer ist, welches auf einem Aktor angeordnet ist, gestaltet für eine lineare und/oder schräge Bewegung.

17. Instrument nach einem oder mehreren der Ansprüche 1 bis 14,
**dadurch gekennzeichnet, dass** das einzelne Instrument eine scharfe Nadel ist, welche auf einem für lineare und/oder schräge Bewegung geregelten Aktor angeordnet ist.

18. Instrument nach einem oder mehreren der Ansprüche 1 bis 14,
**dadurch gekennzeichnet, dass** das einzelne Instrument ein Spreizer ist.

19. Instrument nach einem oder mehreren der Ansprüche 1 bis 14,
**dadurch gekennzeichnet, dass** das einzelne Instrument eine Klammer ist.

20. Instrument nach einem oder mehreren der Ansprüche 1 bis 14,
**dadurch gekennzeichnet, dass** das einzelne Instrument eine Pinzette ist.

21. Instrumentenanordnung umfassend ein Instrument nach einem oder mehreren der Ansprüche 1 bis 8, wobei eine Anzahl identischer Instrumente platziert sind auf einer Instrumentenanordnung, welche sich entlang der Länge der Kanüle, Katheter oder Nadel erstreckt, und wobei die Betätigung des dem Ausgang des Katheters nächsten Instruments angeordnet ist, um ein Instrument von der Instrumentenanordnung freizugeben, und geregelt ist, um es an dem Punkt des Ausgangs des Katheters zur Befestigung des Instruments am/in irgendeiner biologischen Struktur zurückzulassen.

22. Instrumentenanordnung nach Anspruch 21, wobei eine Anzahl identischer Instrumente platziert sind auf einer Instrumentenanordnung, welche sich entlang des Katheters oder der Nadel erstreckt, und wo jedes Instrument angeordnet ist, um individuell betrieben zu werden.

23. Instrumentenanordnung nach Anspruch 21, wobei eine Anzahl nicht identischer Instrumente platziert sind auf einer Instrumentenanordnung, welche sich entlang des Katheters oder der Nadel erstreckt, und wo jedes Instrument angeordnet ist, um individuell betrieben zu werden.

24. Instrumentenanordnung nach irgendeinem der Ansprüche 21 bis 23,
**dadurch gekennzeichnet, dass** das einzelne Instrument eine Klemme ist, welche zum Verbinden biologischen Gewebes oder Gewebebestandteile gestaltet ist, und zum Halten des besagten Gewebes oder Gewebebestandteile angeordnet ist, um Heilung zu ermöglichen.

25. Instrumentenanordnung nach irgendeinem der Ansprüche 21 bis 23,
**dadurch gekennzeichnet, dass** das einzelne Instrument ein ausdehnbares zylindrisches Objekt ist, welches zum Einführen, in einem zusammengezogenen Zustand, in einen biologischen Kanal vorgesehen ist und gestaltet ist, ausgedehnt zu werden, um den besagten Kanal in einem ausgedehnten Zustand zu halten, oder um zwei oder mehr biologischer Kanäle zu verbinden.

## Revendications

1. Outil pour la chirurgie biomédicale, dans lequel :
l'outil comprend un micromuscle polymère stratifié adapté pour induire des changements géométriques et des mouvements via un changement de volume induit par voie électrochimique dans au moins une couche polymère de celui-ci, **caractérisé en ce que**
l'outil est monté sur un support ayant la forme d'une aiguille adaptée pour être insérée dans une canule ou un cathéter, par lequel (laquelle) l'outil peut être électriquement actionné via des moyens externes pour induire un mouvement mécanique pour agir sur une structure biologique.

2. Outil selon la revendication 1, dans lequel le polymère stratifié est constitué par un polymère à couche unique.

3. Outil selon la revendication 1, dans lequel le polymère stratifié est constitué par un polymère bicouche.

4. Outil selon la revendication 1, dans lequel le polymère stratifié est constitué par un polymère multicouche et des couches de métal.

5. Outil selon une ou plusieurs des revendications 1 à 4, dans lequel les micromuscles polymères sont construits par des couches, parmi lesquelles au moins une est un polymère conjugué.

6. Outil selon la revendication 5, dans lequel le polymère conjugué est choisi dans le groupe constitué par des polymères et copolymères de pyrrole, d'aniline, de thiophène, de para-phénylène, de vinylène, et de phénylène, y compris les formes substituées des différents monomères.

7. Outil selon la revendication 1, dans lequel l'outil est construit par un polymère bicouche, où le changement de volume électriquement activé dudit au moins un polymère conjugué est adapté pour entraîner une flexion de ladite bicouche.

8. Outil selon la revendication 1, dans lequel l'outil est construit par un polymère multicouche, où le changement de volume électriquement activé dudit au moins un polymère conjugué est adapté pour entraîner une flexion de ladite multicouche.

9. Outil selon une ou plusieurs des revendications 1 à 8, dans lequel l'outil comprend des moyens destinés à fournir le mouvement mécanique pour le positionnement d'une structure biologique.

10. Outil selon une ou plusieurs des revendications 1 à 8, dans lequel l'outil comprend des moyens destinés à fournir le mouvement mécanique pour le maintien d'une structure biologique.

11. Outil selon une ou plusieurs des revendications 1 à 8, dans lequel l'outil comprend des moyens destinés à fournir le mouvement mécanique pour la découpe d'une structure biologique.

12. Outil selon une ou plusieurs des revendications 1 à 8, dans lequel l'outil comprend des moyens destinés à fournir le mouvement mécanique pour la dilatation d'une structure biologique.

13. Outil selon une ou plusieurs des revendications 1 à 8, dans lequel l'outil comprend des moyens destinés à fournir le mouvement mécanique pour la fortification d'une structure biologique.

14. Outil selon une ou plusieurs des revendications 1 à 8, dans lequel l'outil comprend des moyens destinés à fournir le mouvement mécanique pour l'implantation d'une structure biologique.

15. Outil selon une ou plusieurs des revendications 1 à 14, **caractérisé en ce que** l'outil individuel est une paire de ciseaux.

16. Outil selon une ou plusieurs des revendications 1 à 14, **caractérisé en ce que** l'outil individuel est un couteau, qui est disposé sur un actionneur, étant adapté pour un mouvement linéaire et/ou angulaire.

17. Outil selon une ou plusieurs des revendications 1 à 14, **caractérisé en ce que** l'outil individuel est une aiguille pointue qui est disposée sur un actionneur étant adapté pour le mouvement linéaire et/ou angulaire.

18. Outil selon une ou plusieurs des revendications 1 à 14, **caractérisé en ce que** l'outil individuel est un dilatateur.

19. Outil selon une ou plusieurs des revendications 1 à 14, **caractérisé en ce que** l'outil individuel est une pince.

20. Outil selon une ou plusieurs des revendications 1 à 14, **caractérisé en ce que** l'outil individuel est des brucelles.

21. Ensemble d'outils comprenant un outil selon une ou plusieurs des revendications 1 à 8, dans lequel un certain nombre d'outils identiques sont situés sur un ensemble d'outils s'étendant sur une longueur de la canule, du cathéter ou de l'aiguille, et dans lequel l'actionnement d'un outil le plus proche de la sortie du cathéter est adapté pour libérer un outil de l'ensemble d'outils et est adapté pour le laisser au point de sortie du cathéter pour monter l'outil au niveau d'une/dans une certaine structure biologique.

22. Ensemble d'outils selon la revendication 21, dans lequel un certain nombre d'outils identiques sont situés sur l'ensemble d'outils s'étendant le long du cathéter ou de l'aiguille et où chaque outil est adapté pour être individuellement actionné.

23. Ensemble d'outils selon la revendication 21, dans lequel un certain nombre d'outils non identiques sont situés sur l'ensemble d'outils s'étendant le long du cathéter ou de l'aiguille et où chaque outil est adapté pour être individuellement actionné.

24. Ensemble d'outils selon l'une quelconque des revendications 21 à 23, **caractérisé en ce que** l'outil individuel est une pince adaptée pour joindre des tissus biologiques ou des parties de tissu, et adaptée pour maintenir lesdits tissus ou lesdites parties de tissu pour permettre la cicatrisation.

25. Ensemble d'outils selon l'une quelconque des revendications 21 à 23, **caractérisé en ce que** l'outil individuel est un objet cylindrique extensible conçu pour être inséré, dans un état contracté, dans un tube biologique, et adapté pour être déployé pour maintenir ledit tube dans un état déployé ou pour joindre deux ou plusieurs tubes biologiques.
